Europäisches Patentamt

European Patent Office

Office européen des brevets

(19)

(11) Publication number: **0 341 943**
**A2**

(12) **EUROPEAN PATENT APPLICATION**

(21) Application number: **89304601.1**

(22) Date of filing: **08.05.89**

(51) Int. Cl.4: **A61B 17/22 , A61B 17/36**

(30) Priority: **09.05.88 US 191677**

(43) Date of publication of application:
**15.11.89 Bulletin 89/46**

(84) Designated Contracting States:
**CH DE FR GB IT LI SE**

(71) Applicant: **EASTMAN KODAK COMPANY (a New Jersey corporation)**
**343 State Street**
**Rochester New York 14650(US)**

(72) Inventor: **Helfer, Jeffrey Lawrence c/o EASTMAN KODAK COMP.**
**Patent Department 343 State Street**
**Rochester New York 14650 2202(US)**

(74) Representative: **Davis, Ian Ellison et al**
**Kodak Limited Patent Department Headstone Drive**
**Harrow Middlesex HA1 4TY(GB)**

(54) **Laser assisted atherectomy device and method.**

(57) There are described a surgical catheter (10) and a method that removes stenosis and prevents re-stenosis. The catheter comprises a laser-emitting device (110), most preferably a laser diode, in the distal end (20), whereby optical fibers and their attendant disadvantages are eliminated. Preferably, the diode (110) is coupled with a mechanical cutting edge (56), so that the diode (110) can expose to low power laser light (130) freshly exposed, healthy luminal surfaces (S). Such exposure devitalizes and seals such surfaces to substantially prevent re-stenosis.

FIG.2

## LASER ASSISTED ATHERECTOMY DEVICE AND METHOD

Laser catheters have been known for microsurgery. Such devices commonly include an optical fiber that delivers laser light the length of the catheter, from an exterior source at the proximal end, to an emitting portion of the distal end of the catheter. The exterior source can be a conventional laser of known types, or even a laser diode. Such conventional laser catheters often also include balloons used to properly position the laser-emitting surface at a plaque site, and/or a vacuum line to remove debris. Examples are shown in, e.g., U.S. Patent No. 4,627,436. The aforementioned patent also teaches as a substitute for the laser-light emitting portion, a mechanical cutting edge that severs plaque as the catheter is rotated.

Such devices fail to recognize several disadvantages in their design. The first is that a substantial penalty is paid for the use of optical fibers to deliver laser light the length of a catheter. That is, optical fibers have a risk of power losses as the light is transmitted the fiber length, and outright failure should the fiber crack due to extensive manipulation of the catheter within a patient body. Furthermore, there are substantial power losses and coupling inefficiencies at the external, proximal end. These occur because of laser beam/fiber misalignment and of first-surface reflections. Still further, optical fibers are relatively rigid, increasing the potential for such a catheter to damage the fragile vascular endothelium when advanced through tortuous body vessels.

Second, the use of either a laser or a cutting edge fails to recognize that the use of the two means together provides a substantial synergistic benefit. Stated in other words, a surgical catheter that only mechanically cuts away at plaque often leaves exposed physiologically active vascular tissues capable of initiating a cascade of biological events leading to restenosis. More specifically, such active tissues can and have been found to produce deposition of platelets and subsequent release of platelet-derived growth factors that produce a risk of chronic long-term closure. Representative articles explaining this phenomenon can be found in Am. J. Cardiol., Vol. 60, p.5B - 9B (1987) and Am. J. Cardiol., Vol. 60, p.10B - 16B (1987). Platelet deposition also initiates local generation of thrombin, which subsequently forms the fibrin required to stabilize a growing thrombus or clot, the cause of any acute closure.

But even apart from this, the cutter action described in the 4,627,436 patent, Figures 12-14, suffers the disadvantage of requiring the distal end (and indeed the entire catheter) to rotate to move the cutting edge through the plaque. This in turn means the balloon used to press the cutting edge in place, will scrape the wall of the body vessel. As described in Amer. Heart Journal, Vol. 114, pages 639-643 and especially 642, this scraping action can denude the naturally protective endothelium region and cause new stenoic lesions.

Nor does the use of a laser by itself produce a risk-free result. For one thing, absorption of laser energy tends to generate excessive amounts of heat. If the laser dosimetry is not properly controlled, then excessive damage to healthy underlying tissue can occur. At its extreme this can lead to vessel perforation. That is, the high-powered ablative energy is capable of boring through the vessel wall, a potentially fatal event. Another disadvantage of using laser ablation by itself is that ablation proceeds at a slower rate than removal by cutting, and at a higher investment cost.

Therefore, prior to this invention, there has been a problem of preventing restenosis.

The problem is addressed by a surgical catheter that provides a method for the removal of stenosis in vessels such as blood vessels, that also aids in preventing restenosis.

More specifically, in accord with one aspect of the invention, the above problem is addressed by a surgical catheter comprising a distal end constructed for movement within body vessels of a patient, a proximal end constructed to remain exterior of the patient to allow control of the distal end, a body portion connecting the two ends, the distal end including emitting means for emitting laser light at an energy and in a direction sufficient to treat the sidewalls of the body vessel, and means for generating the laser light; characterized in that the generating means and the emitting means comprise at least one laser diode located at the distal end, and at least one wire extending at least most of the length of the body portion to provide electrical energy to the at least one laser diode.

In accord with another aspect of the invention, the above problem is addressed by a surgical catheter comprising a distal end constructed for movement within body vessels of a patient, a proximal end constructed to remain exterior of the patient to allow control of the distal end, a body portion connecting the two ends, the distal end including means for cutting away occlusive material such as plaque on the interior walls of the patient body vessel, the cutting means including a metallic cutting edge and means for moving it relative to the plaque to be cut; characterized in that the distal end further includes, at a location adjacent to the cutting edge, means for emitting laser light out of the side of the distal end, the emitting means being mounted so as to expose the surface of the body vessel immediately following the passage of the cutting edge over that surface, so that the emitting means is effective to devitalize physiologically active tissues.

In accord with yet another aspect of the invention, the above problem is addressed by a method for removal of stenosis in body vessels and devitalization of physiologically active tissues. The method comprises the steps of

a) inserting into an incision in the patient a surgical catheter comprising a distal end constructed for movement within body vessels of a patient, a proximal end constructed to remain exterior of the patient to allow control of the distal end, a body portion connecting the two ends, the distal end including emitting means for emitting laser light at an energy and in a direction sufficient to treat the sidewalls of the body vessel;

b) removing stenosis at sites on the wall of the body vessel to expose physiologically active, healthy tissue, and

c) immediately thereafter, treating the healthy, active tissue with the laser light to devitalize it.

It is an advantageous feature of the invention that a catheter is provided that quickly removes a stenosis and devitalizes the exposed healthy tissue, all at the same time.

It is another advantageous feature of the invention that the devitalizing function is done with maximum energy coupling efficiency, while at the same time, using a technique that is unlikely to unduly damage the healthy body vessels being treated.

Yet another advantageous feature of the invention is the provision of a laser-emitting catheter that flexes more easily and sustains less power loss than conventional laser catheters.

Still another advantageous feature is to provide such a catheter with a heated cutting edge to more effectively cut soft, fatty tissues.

The present invention will now be described by way of example with reference to the accompanying drawings in which:

Figure 1 is a section view along the axis of the distal end of a catheter constructed in accordance with the invention;

Figure 2 is a section view similar to that of Figure 1, but illustrating the laser diode in operation; and

Figure 3 is a schematic illustration of the use of such a catheter in a patient.

The invention is described hereinafter in connection with its use in cutting away occlusive deposits in an artery, a preferred embodiment. In addition, the invention is useful in catheters used to cut away thickened atheromatous tunica intima within an artery, or other occlusive materials in any other body channel.

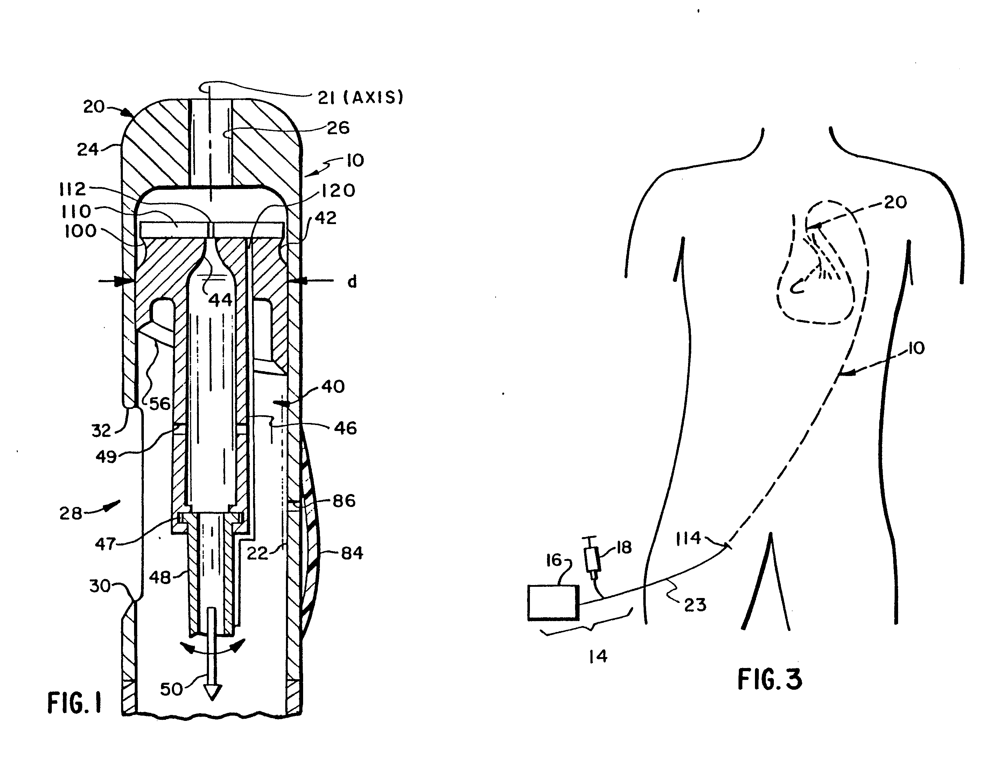

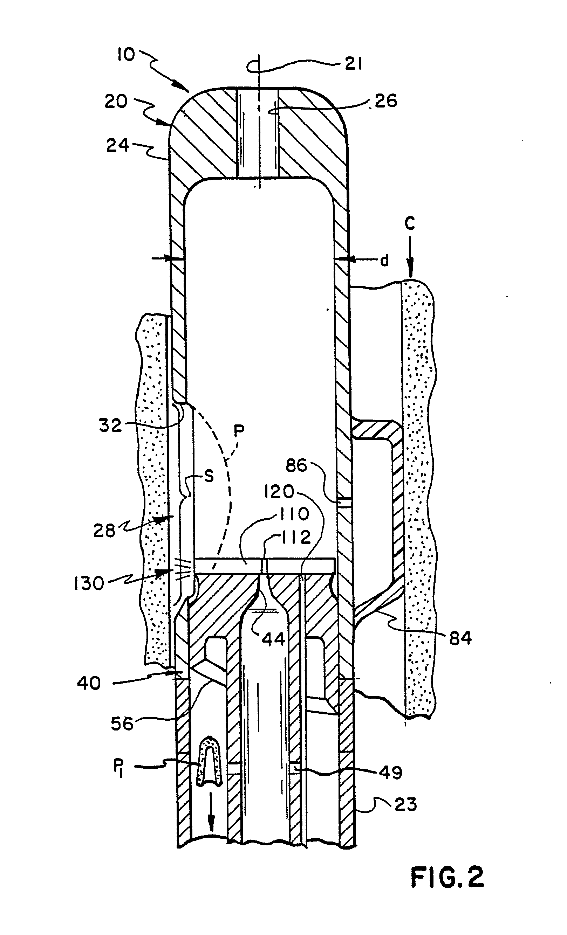

A catheter 10 useful in the practice of the invention comprises a distal end 20 having an axis of symmetry, Figure 1, that is designed for insertion into a body vessel, a proximal control end 14, Figure 3, and a catheter body portion 23, Figures 2 and 3, connecting the two ends. Conventionally, such a catheter is controlled by control means 16, Figure 3.

Distal end 20, Figure 1, comprises a cylindrical tube 22 concentric with axis 21 of the catheter, that is connected in a conventional manner to flexible body 23, Figure 2, and a movable member 40 mounted within tube 22 for movement relative to the tube. To protect the vessel walls while the catheter is advancing through the curvilinear body vessel, tube 22 further preferably comprises shield portion 24 apertured at 26 for additional features hereinafter discussed, and cutting aperture 28. Such aperture has a knife edge 30 that faces forwardly towards the shield portion. Opposite edge 32 of aperture 28 is either a sharp cutting edge, or it can be blunt, since it does not cooperate with the moving member 40 for a shearing action.

Member 40 comprises an end portion 42 having an exterior diameter "d" that permits a sliding fit within the interior walls of cylindrical body 22, and a hollow interior passageway 44. That passageway is part of a sleeve 46 that has a reduced exterior diameter compared to diameter d. Sleeve 46 is then connected at 47 to a hollow tube 48 that extends all the way back through body portion 23 of the catheter. Tube 48 is hollow to allow the flow of, e.g., radiopaque dyes through the catheter and hence through the artery during the operation. Openings 49 are provided in sleeve 46 for aiding in removing cut material. Tube 48 and its connected sleeve 46 and end portion 42 are moved relative to tube 22, (arrow 50) either in a reciprocating fashion, a rotating fashion, or both, by hand or by a conventional, preferably rotating motor in control 16, Figure 3.

To provide shearing action to the distal portion 20, end portion 42 terminates in an annular cutting edge 56 that faces only rearwardly and which cooperates with stationary cutting edge 30 to shear off occlusive deposits that get caught in aperture 28. Edge 56 is the active cutting edge, since without it edge 30 does substantially no cutting, as is intended. Member 40 acts to cut while it is being pulled rearwardly, so that the cut occlusive deposits are directed rearwardly towards body portion 23. To assist in the steady removal of the cut deposits, a wash liquid preferably is pumped towards distal end through tube 48. It exits at apertures 49. (If necessary, aperture 44 can be eliminated to insure flow out of apertures 49.) At the same time, a suction is applied from control 16 to pull liquid and the cut debris down towards the incision, through body portion 23. During this phase of the operation, the patient's blood will not flow through tube 48 or body portion 23, but it is conventional for the catheter diameter to permit blood flow around the outside.

Preferably, a conventional balloon 84 can be mounted on the side of tube 22 opposite to aperture 28, to be expanded by a liquid delivered out through aperture 86 from a feeder line, not shown. Such a balloon is used to push distal end 20 towards an occlusion so that aperture 28 surrounds a portion of it, as is better shown in Figure 2.

Shield aperture 26 can be used for injection of a dye that is sent down the catheter from a syringe 18, Figure 3, through the hollow tube 48, Figure 1.

Most preferably, cutting edge 56 is formed so as to have a spiral locus, measured from end surface 100 of end 42 of member 40, that is continuously variable around the circumference of edge 56. This shape provides greater shearing as member 40 is rotated and withdrawn (arrow 50) relative to tube 22.

(The above-described cutting catheter is described and claimed as such in our copending European Patent Application No. 88303294.8).

In accord with the invention, to insure that the healthy vessel surfaces exposed by the cutting action of edge 56 do not undergo restenosis, a laser-emitting device 110 is mounted on end 100 of member 40, to emit energy sideways that impinges on the freshly exposed surface of the body vessel. As used herein, "healthy" vessel surface or tissue refers to the underlying vessel wall that is substantially free from occlusive deposits, in contrast to those deposits themselves. Any such laser-emitting device is useful, operating at low powers. Preferably, however, to avoid all the disadvantages noted above that are attendant with fiber optics, laser device 110 is a laser diode, thus rendering the catheter free of optical fibers. Such diodes are conventional, and are selected for this invention from miniaturized versions operating at low voltages such as 1.3 volts and low current such as 100-200 milliamps. Preferably a laser diode is used that delivers no more than about 500 milliwatts of power at wavelengths from about 600 nm to about 1000 nm, although other wavelengths are also useful. Such low power is used to ensure that the opposed tissue is devitalized only, and not ablated. However, that power is effective to generate sufficient heat to effect such devitalizing effect. Examples of such preferred diodes include short-wavelength devices radiating at near infra-red wavelengths. Such devices are preferably composed of layers of gallium arsenide (GaAs) and aluminium gallium arsenide (AlGaAs) fabricated on a ceramic substrate. Other semi-conductor materials capable of lasing at other wavelengths are also useful. Any power source can be used to carry power to the diode from the proximal end 14, such as cord 120, Figure 1.

Aperture 112 in diode 110 allows liquid to pass on to aperture 26, Figure 1, from passageway 44.

Figures 2 and 3 illustrate the manner of use of catheter 10. To treat coronary artery disease (for example), distal end 20 is inserted into an incision 114, in a body vessel and worked up the femoral artery, through the aorta until it reaches an occlusion. As this is occurring, member 40 is in the position shown in Figure 1. When fatty build-up or plaque P is located, shown in dashed lines in Figure 2, balloon 84 is inflated to push recess 28 over the plaque. At this point member 40 is pulled rearwardly towards the proximal end, and edge 56 is effective to slice through the plaque. Pieces $P_1$ that have been cut are then pulled through body portion 23 by the vacuum applied at controls 16, or by the rearward motion of the cutting head 40.

As member 40 advances from its position shown in Figure 1, to that shown in Figure 2, power is transmitted to diode 110 to cause it to emit low power laser light, beam 130. This beam is effective to devitalize and seal the freshly exposed surface S, Figure 2, of the vessel wall. As used herein, "devitalize and seal" as applied to the surface exposed by the cutting action of edge 56, refers to the following:

The cutting step exposes healthy, physiologically active vascular tissues prone to acute or chronic closure. Lasing of these tissues acts on medial smooth muscle cells (that proliferate upon exposure to platelet derived growth factors) and destroys them by heating them to a temperature above approximately 48° C - the temperature at which cell death occurs. In addition, the vessel walls are sealed, or thermally coagulated, so as to prevent blood flow to innermost vascular tissues. The result is to retard the proliferation of vascular wall tissues until after re-endothelialization of the excised area has occurred. This in turn aids in substantially preventing restenosis and acute closure.

By mounting device 110 on the metallic member 40, there is provided an automatic heat sink for the diode, preventing excessive heat build up at the diode, and subsequent reduction of energy conversion efficiency. This also acts to heat up the cutting edge, whereby soft, fatty tissue is more easily severed. The heating of the cutting edge is of course at a lower level of magnitude than the heat delivered directly by the laser diode, so that member 40 is not by itself effective to devitalize the healthy vessel tissues.

One of the advantageous features noted above is the maximum energy coupling efficiency in the devitalizing step. This occurs because a) the laser light is created at the distal end by the diode, rather than at the proximal end where power losses occur because of beam misalignment and first-surface reflections. It also occurs because b) the laser light is directed immediately upon the body vessel wall, without the use of an intermediate element that heats up, for example, a balloon that is heated by the laser, as shown in, e.g., EPO Application Publication No. 0182689 (Spears).

Optionally, not shown, more than one laser diode is mounted on end 100, each at a slightly different angle on axis 21, so as to provide a wider angle scan by their combined beams.

Conventional sensing means, not shown, are preferably positioned to sense when diode 110 is facing out of aperture 28 and thus, in position to irradiate the exposed tissue.

**Claims**

1. A surgical catheter comprising a distal end constructed for movement within body vessels of a patient, a proximal end constructed to remain exterior of the patient to allow control of the distal end, a body portion connecting said two ends, said distal end including emitting means for emitting laser light at an energy and in a direction sufficient to treat the sidewalls of the body vessel, and means for generating said laser light;
characterized in that said generating means and said emitting means comprise at least one laser diode located in said distal end, and at least one wire extending at least most of the length of said body portion to provide electrical energy to said at least one laser diode.

2. A surgical catheter comprising a distal end constructed for movement within body vessels of a patient, a proximal end constructed to remain exterior of the patient to allow control of the distal end, a body portion connecting said two ends, said distal end including means for cutting away occlusive material from the interior walls of the patient body vessel, said cutting means including a metallic cutting edge and means for moving it relative to the plaque to be cut;
characterized in that said distal end further includes, at a location adjacent to said cutting edge, means for emitting laser light out of the side of said distal end, said emitting means being mounted so as to expose the surface of the body vessel immediately following the passage of said cutting edge over that surface,
so that said emitting means is effective to devitalize physiologically active tissues.

3. A surgical catheter as defined in claim 2, wherein said emitting means is mounted in contact with said cutting means, whereby said cutting means provide a heat sink for said emitting means.

4. A surgical catheter as defined in claim 2 or 3, wherein said emitting means is a laser diode.

5. A method for removal of stenosis in body vessels and the devitalization of physiologically active tissues, comprising the steps of
a) inserting into an incision in the patient a surgical catheter comprising a distal end constructed for movement within body vessels of a patient, a proximal end constructed to remain exterior of the patient to allow control of the distal end, a body portion connecting said two ends, said distal end including emitting means for emitting laser light at an energy and in a direction sufficient to treat the sidewalls of the body vessel;
b) removing stenosis at sites on the wall of the body vessel to expose physiologically active, healthy tissue, and
c) immediately thereafter, treating said healthy, active tissue with said laser light to devitalize it.

6. A method as defined in claim 5, wherein said laser light is emitted directly onto said tissue at an energy that devitalizes and seals the tissue without ablating it.

7. A method as defined in claim 6, wherein said laser light is emitted from a laser diode mounted in said distal end.

8. A method as defined in claim 5, wherein said removing step comprises the step of moving a mechanical cutting edge relative to the stenosis, said cutting edge being mounted within said distal end adjacent to said emitting means.

9. A catheter as defined in claim 1, 2, 3 or 4, wherein said body portion and said distal end are substantially free of optical fibers.

FIG. 1

FIG. 3

EP 0 341 943 A2

FIG.2